# EUROPEAN PATENT SPECIFICATION

(11) **EP 1 958 655 B1**
(45) Date of publication and mention of the grant of the patent: **06.10.2010**
(21) Application number: 07011829.4
(22) Date of filing: 15.06.2007
(51) Int. Cl.: A61M 5/31

(54) **Syringe barrel and syringe**
Spritzenzylinder und Spritze
Barillet de seringue, et seringue

(30) Priority: 14.02.2007 JP 2007034061
(43) Date of publication of application: 20.08.2008
(73) Proprietor: DAIKYO SEIKO, LTD., Tokyo 131-0031 (JP)
(72) Inventor: Sudo, Masamichi, Tokyo 131-0031 (JP); Kawamura, Hideaki, Tokyo 131-0031 (JP)
(74) Representative: Hartz, Nikolai

(56) References cited:
- EP-A- 1 110 568
- WO-A-99/22788
- DE-A1- 2 108 381
- FR-A- 1 126 718
- US-A- 3 489 147
- US-A- 5 007 903

## Description

### FIELD OF THE INVENTION

This invention relates to a syringe barrel and also to a syringe.

### BACKGROUND OF THE INVENTION

DE-A 21 08 381 discloses a syringe barrel which may have a square, rectangular or pentagonal cross section. A further syringe barrel having a rectangular cross section is known form FR-A 1,126,718.

WO 99/22788 discloses a syringe barrel having a triangular cross section.

Further syringes are known from EP-A 1 110 568, US-A 5,007,903 and US-A 3,489,147.

As syringes for the injection of medical fluid or the aspiration of blood, syringes made of glass or transparent hard resin such as polyethylene resin or polypropylene resin are used widely. Conventionally, it was common to repeatedly use syringes by disinfecting them. In recent years, however, there is a move toward taking more account of safety. These days, many disposable products are used, and prefilled products with medical fluid sealed in syringes are widely available on the market. Examples of medical fluid include those capable of exhibiting effects in extremely small volumes, so that syringes are available in diverse sizes ranging from extremely small sizes to large sizes.

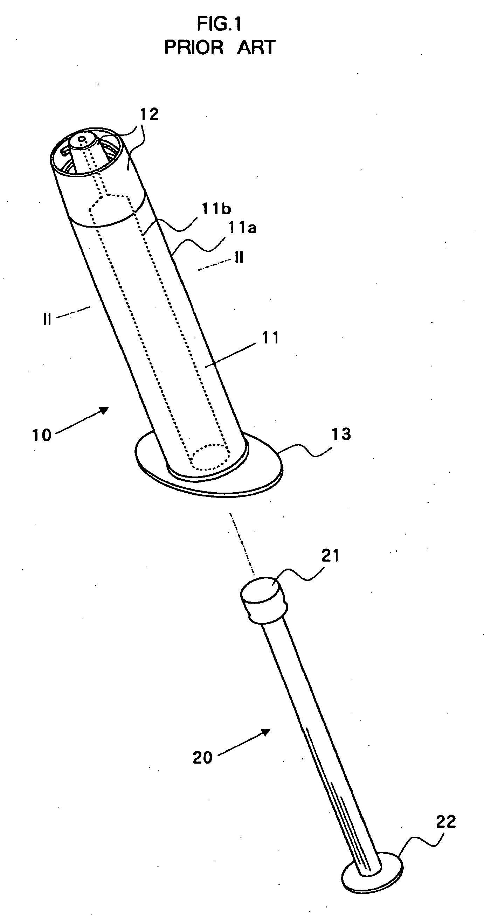

As shown in FIG. 1, a syringe is generally composed of a syringe barrel 10, a plunger 20 slidably insertable into the syringe barrel 10, and a piston 21 secured on a front end of the plunger 20. The syringe barrel 10 is equipped with a hollow barrel body 11 having an outer peripheral wall 11a and an inner peripheral wall 11b, a needle fitting 12 and a finger flange 13. The plunger 20 is provided at its rear end with a thumb rest 22. With respect to the needle fitting 12 and finger flange 13 and also the plunger 20 and piston 21, all of which make up the syringe, various contrivances have been conventionally made to details, and syringes of various shapes are known.

In such conventional syringes, however, the barrel bodies of their syringe barrels each of which accounts for a large majority of the corresponding syringe are all cylindrical. Even if they are different in small details, the overall impressions of the syringes are not different much so that they give substantially the same impression unless they are looked with care. The construction of the barrel body 11 into a cylindrical shape can be attributed especially to the possibility of making an improvement in the close contact between the inner peripheral wall 11b, which defines a hollow space within the barrel body 11, and the piston 21 secured on the front end of the plunge 20 so that medical fluid or the like filled in the syringe can be prevented from leaking out of the syringe, and also to its easier production.

On the other hand, prefilled products with medical fluid sealed in syringes are widely available on the market in recent years as described above. It is the actual circumstances that these prefilled products include prefilled syringes of extremely small volumes while many medical fluids have a similar color. Concerning prefilled syringes with medical fluids of a similar color sealed in syringe barrels of a similar impression, there is a potential risk in some instances that the user could mistake one for the other. In particular, it is not easy to apply labels directly to syringes of extremely small volumes. Therefore, these syringes are often handled in labeled protective cases. There is, however, a potential risk that, once such a syringe is taken out of its labeled protective case, it could be mistaken for another or vice versa. The administration of medical fluid may often be directly vital to the human life. In the development of a syringe, it is, therefore, an extremely important approach to assure the prevention of any erroneous administration of medical fluid.

Because the conventional syringe barrels are cylindrical, they may roll down when placed on tables or the like. During production or use, they may hence be discarded for breakage due to falling. For the cylindrical configurations of the conventional syringe barrels, their outer peripheral walls are smooth, thereby bringing about an advantage that they are excellent in transparency. On the other hand, they are also accompanied by such drawbacks that they may be slippery when held in hand and slight ruggedness, scratches or the like which may be produced on the outer peripheral walls are conspicuous. Accordingly, they may slip down from the hand during use and may be discarded, or during production, they may be found to contain slight ruggedness or the like and may be discarded as being defective in external appearance. Especially in regard to the discard of syringe barrels as defective products although their performance itself has no problem at all, there is a room for improvements from the standpoint of production efficiency. From the current circumstance that more and more disposable products are being used, a still greater reduction is desired in the production cost of syringes themselves by making an increase or the like in material efficiency or production efficiency.

An object of the present invention is, therefore, to provide a syringe barrel, which can provide an impression clearly different from a conventional cylindrical syringe barrel especially when assembled into a prefilled form with medical fluid sealed therein, and also a syringe making use of the barrel. Another object of the present invention is to provide a syringe barrel, which is excellent in handling characteristics, can improve the production efficiency, can save the amount of material to be used and can reduce the production cost, and also a syringe making use of the barrel.

### BRIEF SUMMARY OF THE INVENTION

The above-described objects can be achieved by the present invention to be described hereinafter. In a first aspect of the present invention, there is thus provided a syringe barrel according to claim 1, which is provided with a hollow barrel body, a needle fitting arranged extending in an axial direction from a front end of the barrel body, and a finger flange arranged extending in an outward direction from a rear end of the barrel body, wherein in a cross-section taken at right angles to a central axis of the barrel body, a contour of the barrel body is a specific polygon.

The polygon is selected from the group consisting of a regular hexagon, a regular octagon, a regular decagon and a regular dodecagon. Further, the finger flange can be in a form of a rectangle extending in opposite directions from the rear end of the barrel body as much as from 15 to 30 mm in each direction. In addition, the needle fitting can preferably be a luer lock fitting or a luer tip fitting.

In a second aspect of the present invention, there is also provided a syringe according to claim 5, comprising a syringe barrel, a plunger slidably inserted in the syringe barrel, and a piston secured on the plunger, wherein the syringe barrel is the syringe barrel according to the first aspect of the present invention.

The plunger is provided at a rear end thereof with a thumb rest, and the thumb rest is in a form of a plate extending radially in all directions in a range of from 8 to 15 mm from a central axis of the plunger. The thumb rest has the same polygonal shape as the barrel body.

The syringe according to the present invention can provide an impression clearly different from that of a syringe making use of a conventional cylindrical syringe barrel especially in a state that medical fluid is sealed therein, and therefore, can realize pronounced distinguishability. Further, the syringe barrels according to the present invention and the syringe according to the present invention, which makes use of one of the syringe barrels, are excellent in handling characteristics and good in production efficiency and can reduce the amount of material to be used, and therefore, can lower the production cost.

### BRIEF DESCRIPTION OF THE DRAWINGS

FIG. 1 is a perspective view showing a conventional syringe barrel together with its associated plunger.

FIG. 2 is a cross-sectional view of the conventional syringe barrel taken along line II-II of FIG. 1.

FIG. 3 is a perspective view showing a syringe barrel according to a first embodiment of the present invention together with its associated plunger.

FIG. 4A is a cross-sectional view of a barrel body of the syringe barrel of FIG. 3 taken along line IV A-IV A of FIG. 3, that is, in a plane extending at right angles to a central axis of the barrel body, and FIGS. 4B through 4G are cross-sectional views similar to FIG. 4A and illustrate modifications of the first embodiment, respectively.

FIGS 4E to 4G are not according to the working of the claims.

FIG. 5 is a schematic top plan view for illustrating a step of transporting plural syringe barrels according to the first embodiment of the present invention together while suspending them from plate-shaped hanger members (conveyor).

FIG. 6 is a perspective view showing a syringe barrel according to a second embodiment of the present invention.

### DETAILED DESCRIPTION OF THE INVENTION AND PREFERRED EMBODIMENTS

No particular limitation is imposed on the material of the syringe barrel according to the present invention, and any material can be used insofar as it is usable as a material for the formation of medical devices. Preferred is a material having transparency. Illustrative are glass; and hard resins such as cycloolefin resins, polyethylene resins, polypropylene resins, polyvinyl chloride resins, and polycarbonate resins. Preferred is a hard resin that permits low-cost mass production, and particularly preferred is a cycloolefin resin having properties such as low solubility, high transparency and high gas barrier property.

The syringe barrel according to the present invention, when its material is a hard resin, can be produced by a conventionally-known molding or forming process such as injection molding in a manner commonly employed in the art. In its production, ruggedness or streaks may be formed on the outer peripheral wall of the syringe barrel. Described specifically, it is necessary to inject a resin into a mold upon performing the production by injection molding. A trace of an injection operation of the resin may appear as ruggedness on the outer peripheral wall of the syringe barrel. When a split mold is used as the mold, its mating faces may appear as a raised streak on the outer peripheral wall of the syringe barrel. As a curved surface is formed on an outer peripheral wall in a conventional syringe barrel composed of a circular cylinder, such ruggedness and/or streak is conspicuous. For this shortcoming, limitations have been imposed on the production process. For example, a limitation may be imposed on the location at which resin is to be injected into the mold or, when a split mold is used, a post-processing may be needed. In the syringe barrel according to the present invention, on the other hand, one or more ridges are formed on the outer peripheral wall so that, when the syringe barrel is produced by using such a split mold as having an air vent and mating faces at location(s) corresponding to the ridge(s), such traces can be rendered inconspicuous and the formation of a trace of resin injection can be inhibited. As a result, it is possible to diversify the production process. From this diversification, improvements can be expected in production efficiency.

The syringe according to the present invention is composed of the syringe barrel of the present invention constructed as described above, a plunger slidably insertable into the syringe barrel, and a piston secured on a front end of the plunger. As the plunger and piston which construct the syringe according to the present invention, conventionally-known plungers and pistons are all usable. As the piston, one made of soft resin, elastomer, synthetic rubber or the like, which can provides very close contact with the inner peripheral wall of the syringe barrel, can be used. Most preferred is a piston made of a synthetic rubber and having a film, which is formed of a fluorinated resin excellent in chemical resistance, laminated on the surface of the piston.

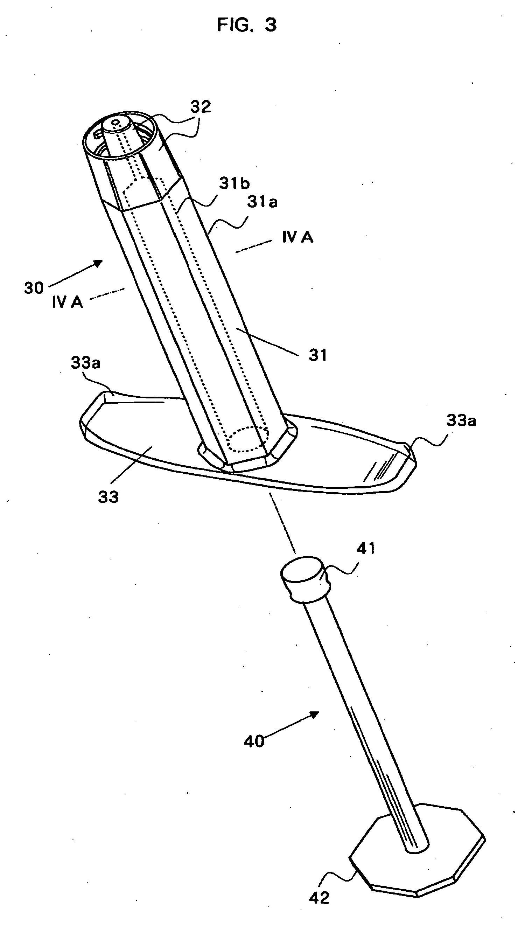

As the plunger, one having a thumb rest at a rear end thereof can be used as in the conventional art. In the present invention, thumb rest is in a form of a plate extending radially in all directions in a range of from 8 to 15 mm from a central axis of the plunger (see FIG. 3). In a conventional thumb rest such as that illustrated in FIG. 1, the area of a portion to which the thumb is brought into contact is small compared with the thickness of the thumb inmany instances so that, upon using the syringe, its holding may appear to lack stability. In the case of the above-described syringe making use of the plunger equipped with the large-diameter thumb rest, on the other hand, the thumb of a user completely fits on the thumb rest upon its use. Its handling characteristics are hence excellent. In the syringe according to the present invention, the thumb rest is configured into a plate-shaped, polygonal form whereby the plate has the same shape as the barrel body. When constructed as described above, the syringe is provided with an excellently balance in external appearance, and further, is possible to more surely avoid the problem that it may roll down from a table or the like when it is placed there.

Preferred embodiments of the present invention will hereinafter be described in detail with reference to the accompanying drawings.

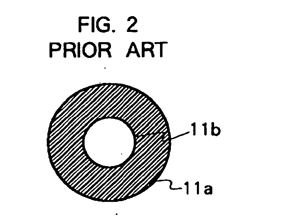

Referring first to FIG. 3, a syringe barrel 30 according to a first embodiment of the present invention will be described. Similar to the conventional syringe barrel shown in FIGS. 1 and 2, the syringe barrel 30 is basically provided with a hollow barrel body 31, a needle fitting 32 arranged extending in an axial direction from a front end of the barrel body 31, and a finger flange 33 arranged extending in an outward direction from a rear end of the barrel body 31. Combined further with a plunger 40 provided with a piston 41 and a thumb rest 42 at its front and rear ends, respectively, a syringe is constructed. In this respect, the syringe barrel 30 is not different at all from the conventional syringe barrel. Moreover, the shapes and constructions of the plunger 40 and piston 41, the shape of a hollow space 31b within the barrel body 31 of the syringe barrel 41 into which the piston 41 can be pushed, and the shape and construction of the needle fitting 32 are, all, not different at all from those of the corresponding elements in the conventional syringe shown in FIG. 1, and can be any corresponding shapes and constructions in the conventionally-known syringes.

The syringe barrel 30 of the first embodiment is characterized in that at least one planar surface section is formed on its outer peripheral wall 31a. Specifically, the surface of the outer peripheral wall 31a is formed in its entirety of plural planar surface sections. Specifically, the barrel body 31 according to the invention is formed such that in a cross-section taken at right angles to a central axis of the barrel body 31, a contour of the barrel body 31 is a specific polygon. By constructing the syringe barrel 30 as described above, planar surface sections and ridges are formed on the outer peripheral wall 31a of the syringe barrel 30 of the first embodiment. As the outer peripheral wall 31a accounts for a large majority of the syringe barrel 30, the syringe barrel 30 is different from the conventional syringe barrel formed of a circular cylinder and having an outer peripheral wall the entire surface of which is formed of a curved surface, and gives a totally different impression from the conventional syringe barrel. As a result, the syringe barrel 30 is provided with outstandingly high distinguishability. Especially when the syringe barrel 30 is applied to a prefilled syringe with medical fluid sealed therein, the syringe barrel 30, owing to the above-described difference in external shape, can hence prevent a user frommistaking another prefilled syringe for the prefilled syringe making use of the syringe barrel 30. Further, the planar surface sections facilitate to apply a sticker and/or a color and/or to write characters. Combined use of such sticker, color and/or characters makes it possible to further enhance the distinguishability. Moreover, the outer peripheral wall 31a is angular, in other words, is multi-sided, leading to improved handling characteristics not only during the production of the syringe barrel but also the use of the syringe. The syringe barrel 30 according to the first embodiment of the present invention with the planar surface sections formed on the outer peripheral wall 31a have various advantages in production and handling as will be mentioned below, and for these advantages, is also useful.

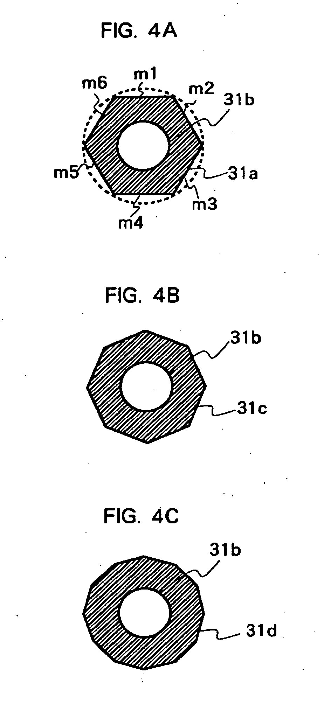

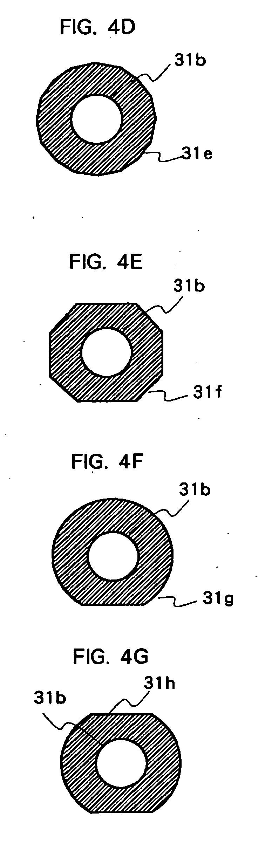

Firstly, the advantages in production will be described hereinafter. FIG. 4A is a cross-sectional view of the barrel body 31 of the syringe barrel 30 according to the first embodiment of the present invention in a plane extending at right angles to the central axis of the barrel body 31. For the sake of a comparison, a cross-sectional view of the barrel body 11 of the conventional syringe barrel 10 made of the circular cylinder in a plane extending at right angles to the central axis of the barrel body 11 (the cross-section along line II-II of FIG. 1) is shown in FIG. 2. The contour of the outer peripheral wall 11a as shown in FIG. 2 corresponds to a dotted circle in FIG. 4A. When the contour of the outer peripheral wall 31a as viewed in its cross-section is, for example, hexagonal as depicted in FIG. 4A, the wall sections on which the planar surface sections are formed, respectively, are smaller in thickness in comparison with the conventional syringe barrel made of the circular cylinder of the same size and indicated by the dotted circle. This means that the material to be used upon production can be reduced, thereby bringing about the advantage that the production cost can be reduced. Moreover, the ridge portions formed on the outer peripheral wall 31a play a bypass-like role for a flow of molten resin during molding and serve to improve the flow of the resin. Compared with the conventional syringe barrel the barrel body of which is formed of the circular cylinder, the syringe barrel 30 of the first embodiment has the advantage that it facilitates thin-wall molding. From the standpoint of this advantage alone, the amount of the material to be used would be reduced further if the barrel body is formed such that the area of one planar surface section becomes wider, specifically if the number of angles is set smaller tomake thin-walled sections wider in the case of a polygon. From the requirements for sufficient gas barrier performance and syringe strength, however, it is necessary to form such planar surface sections while assuring the thin-walled sections to retain an adequate thickness. It is preferred, for example, to design the barrel body such that as the thickness of the thin-walled sections, a thickness of from 0.5 to 4.0 mm or so can be retained.

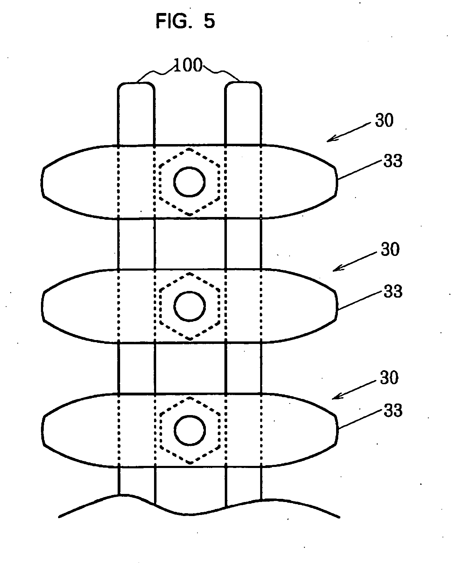

As another advantage in production, the formation of the planar surface sections on the outer peripheral wall 31a of the syringe barrel 30 leads to an improvement in its transportability. FIG. 5 is a schematic view illustrating a step of hooking, between two plate-shaped hanger members (conveyor) 100 disposed in parallel with each other, each syringe barrel 30 at the finger flange 33 arranged on the rear end of the barrel body 31 and transporting a plurality of such syringe barrelstogetherinsuspended positions. With reference to this figure, a description will next be made. When the syringe barrels 30 are to be transported in the above-described manner and the outer peripheral wall 31a of each syringe barrel 30 is provided, for example, with a combination of mutually-parallel planar surfaces, the syringe barrels 30 are all hooked side by side with the same orientation between the above-described two plate-shaped hanger members (conveyor) 100 disposed in parallel with each other, and are then stably transported while maintaining their positions and orientation. In the case of conventional syringe barrels the barrel bodies of which are made of circular cylinders, on the other hand, the syringe barrels are allowed turn in the course of their transportation, and the adjacent finger flanges may overlap each other or the syringe barrels may be transported with varied intervals between them. These shortcomings may become a cause of a reduction in production efficiency in some instances. As readily appreciated from the foregoing, the syringe barrel 30 according to the first embodiment of the present invention shows excellent transportability in the syringe production process owing to its characteristic external shape, and as a consequence, contributes to an improvement in the production efficiency of syringes.

In the syringe barrel according to the first embodiment of the present invention, ridges are formed on and along both side edges of each planar surface section. When the syringe barrel is placed on a table or the like, the angles at these ridges act as resistance to rolling, thereby bring about an advantageous effect that the syringe barrel can be more effectivelyinhibitthe occurrence of troublesome roll-down than the conventional syringe barrel made of the circular cylinder. The troublesome roll-down is considered to more readily take place when the finger flange of the syringe barrel is in the form of a disc. In such a case, these ridges are particularly effective.

When the syringe barrel is produced with a resin, small ruggedness or streaks may be formed on the outer peripheral wall of the barrel body of the syringe barrel. Compared with the conventional syringe barrel that the surface of the outer peripheral wall of its barrel body is formed in its entirety of the curved surface, the syringe barrel according to the first embodiment of the present invention has the advantage that such ruggedness or streaks formed the surface of its barrel body are less conspicuous.

A description will hereinafter be made by taking, as examples, particularly preferred syringe barrels according to the present invention, which are each constructed such that in a cross-section taken at right angles to the central axis of the barrel body, a contour of the barrel body is a polygon. In this construction, the polygon is a regular polygon (see FIG. 4A showing the first embodiment of the present invention, and FIGS. 4B to 4D illustrating modifications of the first embodiment, respectively). A polygon greater in the number of angles than a hexadecagon (see FIG. 4D) is closer to a circle, thereby making it difficult to bring about the above-mentioned advantageous effect that the amount of the material to be used. It is to be noted that in FIGS. 4B through 4G, the outer peripheral walls of the respective modifications are indicated at numerals 31c, 31d, 31e, 31f, 31g and 31h, respectively.

As also mentioned above, it is preferred, for the production of a syringe barrel of excellent transportability, to construct such that the planar surface sections formed on the outer peripheral wall 31a of the syringe barrel 30 include at least one combination of mutually-parallel planar surface sections. In this construction, the profile of the barrel body 31 in a cross-section extending at right angles to the central axis of the barrel body 31 includes at least one combination of mutually-parallel line segments. When the profile is a regular polygon having an even number of angles, for example, as illustrated in FIGS. 4A to 4D, the above-described requirement can be met. A specific example of such one combination of mutually-parallel line segments is a side m1 and a side m4, a side m2 and a side m5, or a side m3 and a side m6. However, one configured to have no specific direction is particularly preferred from the standpoint of production efficiency because its obviates the need of taking the direction into consideration in the production process.

From the foregoing in view, the polygon in the present invention is a regular hexagon (see FIG. 4A), a regular octagon (see FIG. 4B), a regular decagon (not shown), or a regular dodecagon (see FIG. 4C), with a regular hexagon or regular octagon being particularly preferred. A syringe barrel constructed as described immediately above has an external appearance equipped with no specific direction, geometrically well-balanced and high distinguishability, and in addition, is expected to inhibit the occurrence of troublesome roll-down and also to be equipped with improved handling characteristics such as improved holding ease. In the production aspect, on the other hand, improvements are expected in production efficiency, such as improved transportability, improved handling characteristics during production, and a reduction in the amount of the material to be used.

A description will now be made about other elements which also construct the syringe barrel 30 according to the first embodiment of the present invention. The finger flange 33, which is arranged extending in the outward direction from the rear end of the barrel body 31, will be described firstly. A shape of the finger flange 33, said shape being suited in the present invention, is depicted in FIG. 3. Nonetheless, the present invention is not limited to such a shape, and the shape or construction of the finger flange 33 can be designed following the definition of claim 1. The finger flange 33 exemplified in FIG. 3 is substantially in the form of a rectangle extending in opposite directions from the rear end of the barrel body 31 as much as from 15 to 30 mm in each direction, and is equipped with such advantages as will be described below. A conventionally-known finger flange is designed in the form of an ellipse outwardly extending a little in opposite directions from a rear end of a barrel body (see FIG. 1) or in the form of a circle extending a little in all directions from a rear end of a barrel body. In such a conventionally-known finger flange, the length of the extending part is short compared with the thickness of the middle and index fingers. When a syringe equipped with such a finger flange is used, the positions of the middle and index fingers may not be stable depending on the user. When the flange is in a substantially rectangular form as described above, on the other hand, the lengths extending in the opposite directions are either substantially equal or slightly longer than the thickness of the fingers. When the syringe barrel 30 equipped with the finger flange 33 of the above-described substantially rectangular form is used as a syringe, the middle and index fingers, therefore, firmly fit on the finger flange 33 so that a sensation of high stability is obtained. In addition, the above-described substantially rectangular finger flange 33 may preferably be provided with raised portions 33a on front walls of is opposite ends (see FIG. 3). Also preferred is a substantially rectangular finger flange with gentle convexities, more specifically upwardly arcuate convexities formed on the finger flange at areas where the fingers are brought into contact with the finger flange. When constructed as described above, the middle and index fingers more firmly fit on the finger flange 33 so that the syringe can be used in a more stable state.

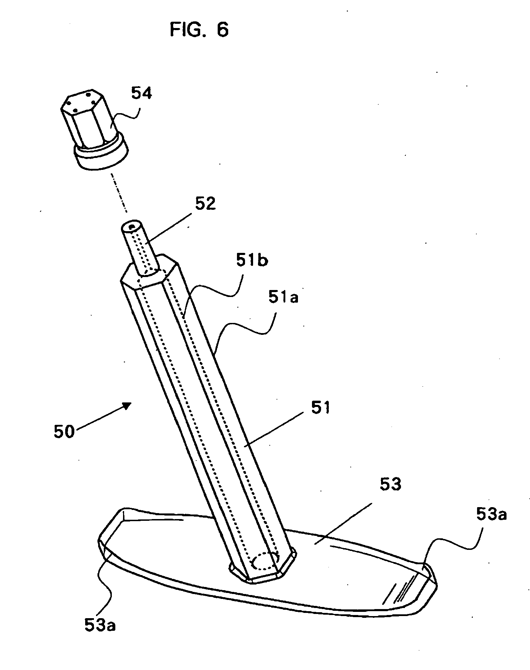

The syringe barrel 30 according to the first embodiment of the present invention is equipped with the needle fitting 32, which is arranged extending in the axial direction from the front end of the barrel body 31 and is used to fit an injection needle. No particular limitation is imposed on the shape, size or position of the needle fitting 32, and conventionally-known needle fittings are all usable. As the needle fitting 32, a luer lock fitting exemplified in FIG. 3 or a luer tip fitting exemplified in FIG. 6 can be used in the present invention.

Referring next to FIG. 6, a syringe barrel 50 according to a second embodiment of the present invention will be described. The syringe barrel 50 is provided with a hollow barrel body 51, a needle fitting 52 arranged extending in an axial direction from a front end of the barrel body 51, a nozzle cap 54 detachably fitted on the needle fitting 52 to prevent medical fluid from flowing out from the needle fitting 52, and a finger flange 53 arranged extending in an outward direction from a rear end of the barrel body 51. As described above, the needle fitting 52 in the second embodiment is formed as a luer tip fitting. It is to be noted that an outer peripheral wall 51a and inner peripheral wall 51b of the barrel body 51 and raised portions 53a formed on opposite ends of the finger flange 53 are similar to the corresponding elements in the syringe barrel 30 of the first embodiment shown in FIGS. 3 to 5 and their description is omitted herein.

While a syringe making use of the syringe barrel 50 of the second embodiment is not in use, the nozzle cap 54 is kept fitted on the needle fitting 52 to prevent medical fluid from flowing out. As the nozzle cap 54, conventionally-known nozzle caps are all usable. However, preferred is a nozzle cap configured such that at least a part of its contour in a cross-section extending at right angles to its central axis is polygonal. This nozzle cap is provided with an improved balance in external appearance and hence, can enjoy higher distinguishability from other products. As an example, a shape composed in combination of a circular cylinder and a hexagonal cylinder as in FIG. 6 can be mentioned. No particular limitation is imposed on the material of the nozzle cap 54, and a material similar to that mentioned above for the piston can be used.

## Claims

1. A syringe barrel (30; 50) provided with a hollow barrel body (31; 51) defining a hollow space (31b) therethrough to permit insertion of a plunger therein, a needle fitting arranged extending in an axial direction from a front end of said barrel body, and a finger flange (33; 53) arranged extending in an outward direction from a rear end of said barrel body wherein:
(i) said finger flange is substantially rectangular and has raised portions (33a; 53a) formed on opposing ends of the finger flange; and
(ii) in a cross-section taken at a right angle to a central axis of said barrel body, a contour of said barrel body is a polygon selected from the group consisting of a regular hexagon, a regular octagon, a regular decagon and a regular dodecagon, and a contour of said hollow space (31 b) is a circle.

2. A syringe barrel according to claim 1, wherein said finger flange (33;53) is in a form of a rectangle extending in opposite directions from said rear end of said barrel body (31;51) as much as from 15 to 30 mm in each direction.

3. A syringe barrel according to claim 1, wherein said needle fitting is a Luer lock fitting (32).

4. A syringe barrel according to claim 1, wherein said needle fitting is a Luer tip fitting (52).

5. A syringe comprising
(a) a syringe barrel,
(b) a plunger (40) including a plunger head (41) secured an a front end of said plunger (40), said plunger head (41) being slidably inserted in said syringe barrel, wherein:
- said syringe barrel is a syringe barrel (30;50) as defined in any one of claims 1 to 4, and
- said plunger (40) is provided at a rear end thereof with a thumb rest (42), and said thumb rest (42) is in a form of a plate extending radially in all directions in a range of from 8 to 15 mm from a central axis of said plunger (40) and having the same shape as said barrel body.

## Patentansprüche

1. Spritzenschaft (30; 50) mit einem hohlen Schaftkörper (31; 51), der einen hohlen Raum (31 b) durch diesen definiert, um eine Einführung eines Kolbens in diesen zu gewährleisten, ein Nadelanschlussstück, das angeordnet ist, sodass es sich in axialer Richtung von einer Vorderseite des Schaftkörpers erstreckt, sowie ein Fingerflansch (33; 53), der angeordnet ist, sodass er sich nach außen gerichtet vom hinteren Ende des Schaftkörpers erstreckt, wobei:
(i) der Fingerflansch im wesentlichen rechteckig ist und erhöhte Bereiche (33a; 53a) aufweist, die an gegenüberliegenden Enden des Fingerflansches ausgebildet sind; und
(ii) in einem Querschnitt, der im rechten Winkel zur Mittelachse des Schaftkörpers verläuft, der Umriss des Schaftkörpers ein Polygon ist, das ausgewählt wird, aus der Gruppe, bestehend aus einem gleichseitigen Hexagon, einem gleichseitigen Octagon, einem gleichseitigen Decagon und einem gleichseitigen Dodecagon, und ein Umriss des Hohlraums (31 b) ein Kreis ist.

2. Der Spritzen schaft nach Anspruch 1, wobei der Fingerflansch (33; 53) in Form eines Rechtecks vorliegt, das sich in gegenüberliegenden Richtungen von dem hinteren Ende des Schaftkörpers (31; 51) bis zu 15 bis 30 mm in jede Richtung erstreckt.

3. Der Spritzenschaft nach Anspruch 1, wobei das Nadelanschlussstück eine Luer-Lock-Anschlussstück (32) ist.

4. Der Spritzenschaft nach Anspruch 1, wobei das Nadelanschlussstück ein Luer-Spitzenanschlussstück (52) ist.

5. Spritze, umfassend
(a) einen Spritzenschaft,
(b) einen Kolben (40), der einen Kolbenkopf (41), der an der Vorderseite des Kolbens (40) befestigt ist, aufweist, wobei der Kolbenkopf (41) gleitend in den Spritzenschaft eingeführt werden kann, wobei:
- der Spritzenschaft ein Spritzenschaft (30; 50), wie er in einem der Ansprüche 1 bis 4 definiert wird, ist, und
- Der Kolben (40) an dessen Rückseite mit einer Daumenauflage (42) vorgesehen wird, und die Daumenauflage (42) in Form einer Platte vorliegt, die sich radial in alle Richtungen in einem Bereich von 8 bis 15 mm von einer Mittelachse des Kolbens (40) erstreckt und dieselbe Form wie der Spritzenkörper aufweist.

## Revendications

1. Tube de seringue (30 ; 50) pourvu d'un corps de tube creux (31 ; 51) qui définit un espace creux (31b) pour permettre d'y introduire un piston, d'un raccord d'aiguille qui s'étend dans un sens axial à partir d'une extrémité avant du corps de tube, et d'une collerette pour les doigts (33 ; 53) qui s'étend vers l'extérieur à partir d'une extrémité arrière du corps de tube, étant précisé :
(i) que la collerette pour les doigts est globalement rectangulaire et a des parties relevées (33a ; 53a) formées sur ses extrémités opposées ; et
(ii) qu'en section transversale réalisée à angle droit par rapport à un axe central du corps de tube, un contour dudit corps de tube a une forme polygonale choisie dans le groupe comprenant un hexagone régulier, un octogone régulier, un décagone régulier et un dodécagone régulier, et un contour de l'espace creux (31b) est circulaire.

2. Tube de seringue selon la revendication 1, dans lequel la collerette pour les doigts (33 ; 53) a la forme d'un rectangle qui s'étend dans des directions opposées à partir de l'extrémité arrière du corps de tube (31 ; 51), de 15 à 30 mm dans chaque direction.

3. Tube de seringue selon la revendication 1, dans lequel le raccord d'aiguille est constitué par un raccord Luer lock (32).

4. Tube de seringue selon la revendication 1, dans lequel le raccord d'aiguille est constitué par un raccord Luer tip (52).

5. Seringue comprenant
(a) un tube de seringue,
(b) un piston (40) présentant une tête de piston (41) fixée à une extrémité avant du piston (40), la tête de piston (41) étant glissée dans le tube de seringue, étant précisé :
- que le tube de seringue est constitué par un tube de seringue (30 ; 50) tel qu'il est défini dans l'une quelconque des revendications 1 à 4 ; et
- que le piston (40) est pourvu, à son extrémité arrière, d'un appui pour le pouce (42), et cet appui pour le pouce (42) a la forme d'une plaque qui s'étend radialement dans toutes les directions, sur une distance de 8 à 15 mm, à partir d'un axe central du piston (40) et qui a la même forme que le corps de tube.
